# EUROPÄISCHE PATENTSCHRIFT

(11) **EP 0 354 943 B1**
(45) Veröffentlichungstag und Bekanntmachung des Hinweises auf die Patenterteilung: **29.12.1993**
(21) Anmeldenummer: 89901555.6
(22) Anmeldetag: 19.01.1989
(51) Int. Cl.: A61B 17/16, B25D 9/02

(54) **SCHLAGWERKZEUG FÜR CHIRURGISCHE INSTRUMENTE**
PERCUSSION TOOL FOR SURGICAL INSTRUMENTS
OUTIL PERCUTANT POUR INSTRUMENTS CHIRURGICAUX

(30) Priorität: 21.01.1988 DE 3801676; 25.01.1988 DE 3802033
(43) Veröffentlichungstag der Anmeldung: 21.02.1990
(73) Patentinhaber: AESCULAP AG, D-78501 Tuttlingen (DE)
(72) Erfinder: APPEL, Hans-Günter, D-2948 Accum (DE); LAABS, W., A., D-2940 Wilhelmshaven (DE); HEMEYER, Thorsten, D-2858 Schiffdorf-Wehdel (DE); HÄUSLER, Rainer, D-7200 Tuttlingen (DE); WÖLFLE, Wilfried, D-7737 Bad Dürrheim (DE)
(74) Vertreter: Hoeger, Stellrecht & Partner
(86) Internationale Anmeldenummer: EP8900059
(87) Internationale Veröffentlichungsnummer: WO8906516

(56) Entgegenhaltungen:
- EP-A- 0 144 005
- DE-C- 3 229 309
- US-A- 3 456 739

## Beschreibung

Die Erfindung betrifft ein Schlagwerkzeug für chirurgische Instrumente mit einem hülsenförmigen Griffteil, einem in Längsrichtung in diesem verschieblichen Werkzeughalter und einem im Griffteil in dessen Längsrichtung verschieblichen, oszillierend angetriebenen Kolben, der zwei Schlagflächen aufweist, die an entsprechenden Anlageflächen des Werkzeughalters anschlagen und diesen dabei mit in entgegengesetzter Richtung wirkenden Kraftstößen beaufschlagen.

Schlagende Werkzeuge werden in der Chirurgie beispielsweise eingesetzt, um Raspeln in den Markraum eines Knochens einzutreiben und damit diesen Markraum für die Aufnahme einer Endoprothese vorzubereiten, oder zum Einsetzen von Prothesenschäften in den Markraum von Knochen. Es ist dabei bekannt, als Schlagwerkzeuge beispielsweise Werkzeuge zu verwenden, die einen oszillierend angetriebenen Schlagkolben aufweisen, der bei jedem Hub seiner Bewegung gegen einen Werkzeughalter schlägt und somit einen Schlag zum Eintreiben des Werkzeugs in den Knochen auf den Werkzeughalter ausübt. Dieser Kolben kann beispielsweise pneumatisch durch entsprechende Umsteuerung von Ventilen oszillierend angetrieben werden.

Es ist weiterhin bekannt, ein solches Instrument so auszubilden, daß auf das in den Knochen eingetriebene Instrument bei jedem Hub des Schlagkolbens nicht nur ein das Instrument in Vorwärtsrichtung beaufschlagender Stoß geführt wird, sondern auch ein in entgegengesetzter Richtung wirkender Stoß, so daß das Instrument bei jedem Hub auch wieder gegenüber dem Knochenraum gelockert wird (EP-B1 144 005). Dies wird bei der bekannten Konstruktion dadurch erreicht, daß der Kolben am Ende seiner Bewegung an einander gegenüberliegenden Anschlagflächen des Werkzeughalters anschlägt und daß das Instrument in axialer Richtung unverschieblich mit dem Werkzeughalter verbunden ist. Dadurch werden bei jedem Hub in entgegengesetzter Richtung wirkende Schläge auf das Instrument übertragen.

Ein solches Instrument ist zum Eintreiben von Raspeln, Prothesenschäften etc. geeignet, jedoch nicht zum Herausziehen derartiger Instrumente, beispielsweise zum Herausziehen einer im Merkraum eines Knochens festsitzenden Raspel.

Andererseits gibt es auf verschiedenen technischen Gebieten zur Anwendung kommende Schlaghämmer, die mit einem fliegenden Kolben arbeiten. Bei einem bekannten pneumatischen Karosseriemeißel ist an die vordere und an die hintere Zylinderkammer je ein Druckluftkanal angeschlossen, die über ein Flatterventil wechselweise mit einer Druckluftquelle verbunden werden. Im vorderen Bereich der hinteren Zylinderkammer ist eine Entlüftungsbohrung angeordnet, die beim Vortrieb zunächst vom fliegenden Kolben verschlossen ist und kurz vor dem Auftreffen auf den axial verschiebbar gelagerten Meißel geöffnet wird, wobei der vor dem Kolben entstehende Staudruck durch ein Spiel zwischen dem Meißelbolzen und der Führungsbohrung im Gehäuse entweicht. Sobald die Entlüftungsbohrung freiliegt, stellt sich das Flatterventil infolge des Druckabbaus um, so daß nun die vordere Zylinderkammer mit Druckluft beaufschlagt wird und den Kolben nach hinten treibt, das heißt vom Werkzeug weg. Wird nun das Entlüftungsventil vom Kolben wieder verschlossen, baut sich in der hinteren Zylinderkammer wieder ein Druck auf, der schließlich das Zweiwegeventil umsteuert und den nächsten Vorwärtshub einleitet. Bei dieser Ausführung schlägt der fliegende Kolben nicht auf das Zylindergehäuse. Für medizinische Zwecke ist das Gerät aber vor allem deshalb nicht brauchbar, weil das Werkzeug nur in Richtung zum Objekt hin beaufschlagt wird, eine Raspel also nicht herausziehbar wäre.

Weiterhin ist aus der DE 32 29 309 C 2 ein hydraulischer Schlaghammer bekannt, bei dem ein fliegender Hohlkolben vorgesehen ist, in dem ein auf ein Werkzeug einwirkender, als Kolben ausgebildeter Schlagbolzen axial verschiebbar gelagert ist. Mittels einer bestimmten Führung des hydraulischen Mediums und eines Zweiwegeventils werden der Hohlkolben und der kolbenartige Schlagbolzen wechselweise gegen einen am Werkzeug anliegenden Zwischenkolben getrieben. Dieses Gerät arbeitet nur, wenn das Werkzeug gegen das Objekt gedrückt wird. eine medizinische Anwendung im Operationsbereich scheidet auch hier aus.

Zur Knochenchirurgie gehört weiterhin das Ein- und Austreiben von Marknägeln, das Verdichten von Knochengewebe und Knochenzement und das Bearbeiten von Knochen mit einem Meißel. Diese Maßnahmen werden bisher ebenfalls ausschließlich manuell mit Hilfe eines Hammers vorgenommen.

Es ist Aufgabe der Erfindung, ein gattungsgemäßes Schlagwerkzeug derart zu verbessern, daß es von der Bedienungsperson ohne Umrüstarbeiten wahlweise zum Einschlagen und zum Herausziehen eines Instrumentes eingesetzt werden kann.

Diese Aufgabe wird bei einem Schlagwerkzeug der eingangs beschriebenen Art erfindungsgemäß dadurch gelöst, daß der tatsächliche Hub des Kolbens durch Anschläge im Griffteil derart beschränkt ist, daß er kleiner ist als der Doppelschlaghub, den der Kolben bei im Griffteil festgehaltenem Werkzeughalter vom Anschlagen der ersten Schlagfläche an der ersten Anlagefläche bis zum Anschlagen der zweiten Schlagfläche an der zweiten Anlagefläche zurücklegen müßte, und daß der Werkzeughalter sowohl relativ zum Griffteil als auch relativ zum Kolben verschieblich im Griffteil gelagert ist.

Ein solches Schlagwerkzeug überträgt auf das in ihm gehaltene Instrument nur nach vorne gerichtete Schläge, wenn die Bedienungsperson den Griffteil in Einschlagrichtung gegen den Knochen drückt, es überträgt dagegen das I..strument aus dem Knochen heraustreibende Schläge auf das Instrument, wenn die Bedienungsperson den Griffteil vom Knochen wegzieht. Dies ergibt sich dadurch, daß durch die Begrenzung des Kolbenhubes bei einer Hubbewegung des Kolbens immer nur entweder die eine oder die andere Schlagfläche des Kolbens an der entsprechenden Anlagefläche des Werkzeugs anschlagen kann, es ist aber ausgeschlossen, daß während eines Hubes beide Schlagflächen an der jeweils anderen Anlagefläche anschlagen. Welche der beiden Schlagflächen an ihrer Anlagefläche anschlägt, läßt sich durch die Position der Anlageflächen des Werkzeuges innerhalb des Hubweges des Kolbens bestimmen. Dieser Hubweg des Kolbens, der durch den Aufbau des Griffteils definiert wird, läßt sich relativ zum Werkzeug dadurch verschieben, daß der Griffteil gegenüber dem Knochen verschoben wird, in dem das Werkzeug gehalten ist. Der Operateur kann also einfach durch Drücken oder Schieben des Griffteils die Richtung der Schlagwirkung umdrehen, wobei die tatsächliche Bewegung des Griffteils beim Wechsel der Kraftrichtung gering sein kann, beispielsweise kann es genügen, den Griffteil gegenüber dem Werkzeug um 1 mm zu verschieben, um eine Umkehr der Schlagrichtung zu erreichen.

Diese Umkehrmöglichkeit der Schlagrichtung gibt dem Operateur auch während des Einsetzens eines Prothesenschaftes die Möglichkeit, die Prothese nach dem stückweisen Einschlagen kurzzeitig wieder etwas zu lockern, um eine Überprüfung des Sitzes oder eine Korrektur der Einschlagrichtung vorzunehmen. Dazu müssen keinerlei Umstellungen an dem Werkzeug selbst vorgenommen werden, so daß eine solche Umkehr auch außerordentlich gefühlvoll erfolgen kann.

Bei einer bevorzugten Ausführungsform ist vorgesehen, daß der Kolben einen Innenraum aufweist, in den durch eine Stirnfläche des Kolbens hindurch der Werkzeughalter eintritt, daß die in entgegengesetzte Richtung weisenden Anlageflächen des Werkzeughalters im Innenraum des Kolbens angeordnet sind, und daß sich auch die Schlagflächen des Kolbens in diesem Innenraum befinden. Dadurch ergibt sich eine besonders platzsparende Anordnung.

Es ist dabei vorteilhaft, wenn der Werkzeughalter an seinem im Inneren des Kolbens angeordneten Ende einen Ringflansch trägt, dessen gegenüberliegende Stirnflächen die beiden Anlageflächen bilden.

Weiterhin können die den Innenraum des Kolbens begrenzenden Stirnflächen die beiden Schlagflächen bilden.

Es kann genügen, wenn der Hub des Kolbens nur um etwa 1 mm kleiner ist als der Doppelschlaghub, also der Hub, bei dem beide Schlagflächen an den entsprechenden Anlageflächen anschlagen würden. Dadurch ergibt sich eine geringe Bauhöhe des Instruments und außerdem eine sehr feinfühlige Richtungumkehr der Schlagwirkung.

Damit das pneumatische Schlagwerkzeug wahlweise mit verschiedenen Werkzeugen ausgerüstet werden kann, ist das Werkzeug zweckmäßig durch einen Bajonettverschluß oder dergleichen lösbar mit dem Schlagbolzen verbunden.

Die nachfolgende Beschreibung bevorzugter Ausführungsformen der Erfindung dient im Zusammenhang mit der Zeichnung der näheren Erläuterung. Es zeigen:
- Figur 1 :: eine Längsschnittansicht eines raspelförmigen Schlagwerkzeugs in Ausgangsstellung ;
- Figur 2 :: eine schematische Darstellung der Kolbenstellung und der Werkzeugstellung beim Einschlagen des Werkzeugs und bei zurückgezogenem Kolben;
- Figur 3 :: eine Ansicht ähnlich Figur 2 beim Einschlagen des Werkzeugs mit dem Kolben in Einschlagposition;
- Figur 4 :: eine Ansicht ähnlich Figur 2 beim Herausziehen des Werkzeugs mit dem Kolben in Ausziehposition und
- Figur 5 :: eine Ansicht ähnlich Figur 4 beim Herausziehen des W rkzeugs mit vorgeschebenem Kolben.

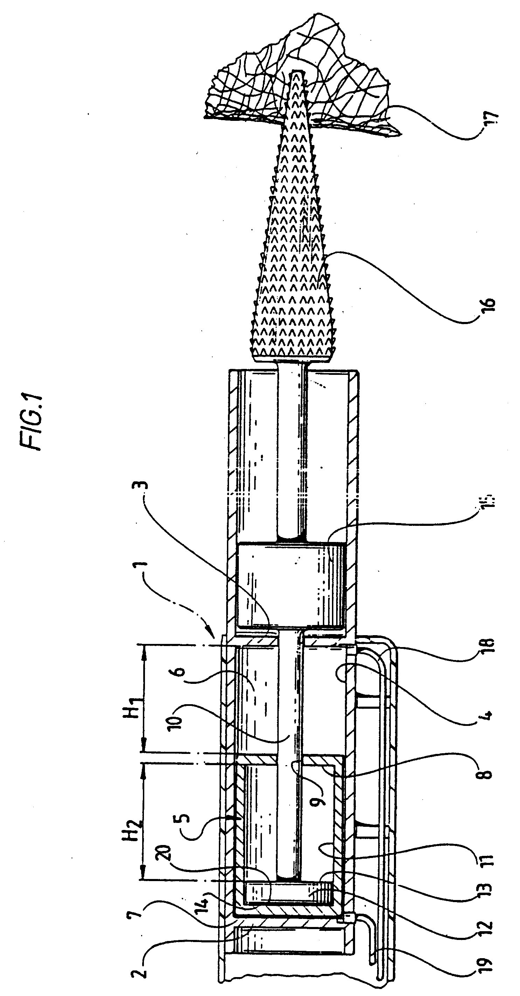

Das in den Figuren 1 bis 5 dargestellte Schlagwerkzeug umfaßt einen hülsenförmigen Griffteil 1 mit einem beidseitig durch Wände 2 und 3 abgeschlossenen Zylinderraum 4, den ein in dem Zylinderraum 4 abgedichtet und verschieblich gelagerter Kolben 5 in eine vordere Kammer 6 und in eine hintere Kammer 7 unterteilt. Der Kolben 5 ist hohl ausgebildet und weist in seiner vorderen Stirnwand 8 eine zentrale Öffnung 9 auf, durch die eine Stange 10 in den Innenraum 11 des Kolbens 5 eintritt. Diese Stange 10 trägt an ihrem freien, im Innenraum 11 angeordneten Ende einen Ringflansch 12, dessen der Stange 10 zugewandte Fläche eine vordere Anschlagfläche 13 bildet, während die gegenüberliegende Stirnfläche des Ringflansches 12 eine hintere Anschlagfläche 14 bildet.

Die Stange 10 tritt abgedichtet auch durch die vordere Wand 3 des Zylinderraumes 4 hindurch und ist dort einstückig mit einer im Griffteil 1 längsverschieblich gelagerten Werkzeughalterung 15 verbunden, in die in nicht näher dargestellter Weise ein Instrument eingesetzt ist, beispielsweise eine sich zum vorderen Ende hin verjüngende Raspel 16, die zur Aufweitung des Markraumes eines Knochens 17 verwendet wird. Das Werkzeug ist in der Werkzeughalterung 15 in axialer Richtung unverschieblich gehalten und kann durch weitere in der Zeichnung nicht näher dargestellte Führungsmittel in Längsrichtung im Griffteil geführt sein.

Sowohl in die vordere Kammer 6 als auch in die hintere Kammer 7 mündet je eine Druckmittelleitung 18 beziehungsweise 19 ein, die durch eine im hinteren, in der Zeichnung nicht dargestellten Bereich des Griffteils 1 angeordneten Steuerung abwechselnd mit einer Druckluftquelle verbunden werden, während zusätzliche Entlüftungsventile vorgesehen sein können, die die jeweils nicht mit der Druckmittelquelle verbundene Kammer des Zylinderraumes 4 belüften. Diese Entlüftungsventile sind in der Zeichnung nicht dargestellt. Derartige pneumatischen Antriebe zur periodisch oszillierenden Bewegung eines Kolbens in einem Zylinderraum sind an sich bekannt und werden daher hier nicht ausführlich erläutert.

Die periodische Hubbewegung des Kolbens 5 im Zylinderraum 4 wird begrenzt durch die als Anschlag wirkenden Wände 2 und 3, so daß sich ein maximaler Hub des Kolbens 5 im Zylinderraum 4 aus der Differenz des Abstandes zwischen den beiden Wänden 2 und 3 und der axialen Länge des Kolbens 5 ergibt. Der maximale Hub des Kolbens 5 im Zylinderraum 4 ist in Figur 1 mit H₁ bezeichnet.

Der Kolben 5 ist gegenüber der in seinem Innenraum 11 hineinragenden Stange 10 frei verschieblich. Die Wegstrecke dieser Relativverschiebung wird durch die Position der Anschlagflächen 13 und 14 sowie die Position der den Innenraum begrenzenden vorderen Stirnwand 8 und der gegenüberliegenden hinteren Stirnwand 20 des Innenraums 11 bestimmt. Der Hub dieser Relativverschiebung ist in Figur 1 mit H₂ bezeichnet. H₂ bezeichnet den Hub, den die Stange 10 mit dem Ringflansch 12 relativ zum Kolben 5 ausführen müßte, um ein Anschlagen des Ringflansches 12 sowohl an der vorderen Stirnwand 8 als auch an der hinteren Stirnwand 20 zu erreichen. Dieser Hub wird im folgenden als Doppelschlaghub bezeichnet, denn wenn der Kolben 5 einen Hub ausführt, der so groß ist wie dieser Doppelschlaghub oder größer, dann schlägt der Kolben bei beiden Bewegungsrichtungen gegen den Ringflansch 12 und überträgt somit bei jeder Bewegungsperiode sowohl einen nach vorne gerichteten als auch einen nach hinten gerichteten Schlag auf den Ringflansch 12 und die damit verbundene Werkzeughalterung.

Erfindungsgemäß wird jedoch der Hub des Kolbens 5 so dimensioniert, daß er kleiner ist als der Doppelschlaghub H₂. Dies hat zur Folge, daß bei einer Periode der Kolbenbewegung der Kolben nur entweder an der vorderen oder an der hinteren Anlagefläche 13 beziehungsweise 14 des Ringflansches 12 anschlagen kann, das heißt bei jeder Periode des Kolbenhubes wird nur ein entweder nach vorne oder nach hinten gerichteter Schlag auf die Werkzeughalterung 15 ausgeübt.

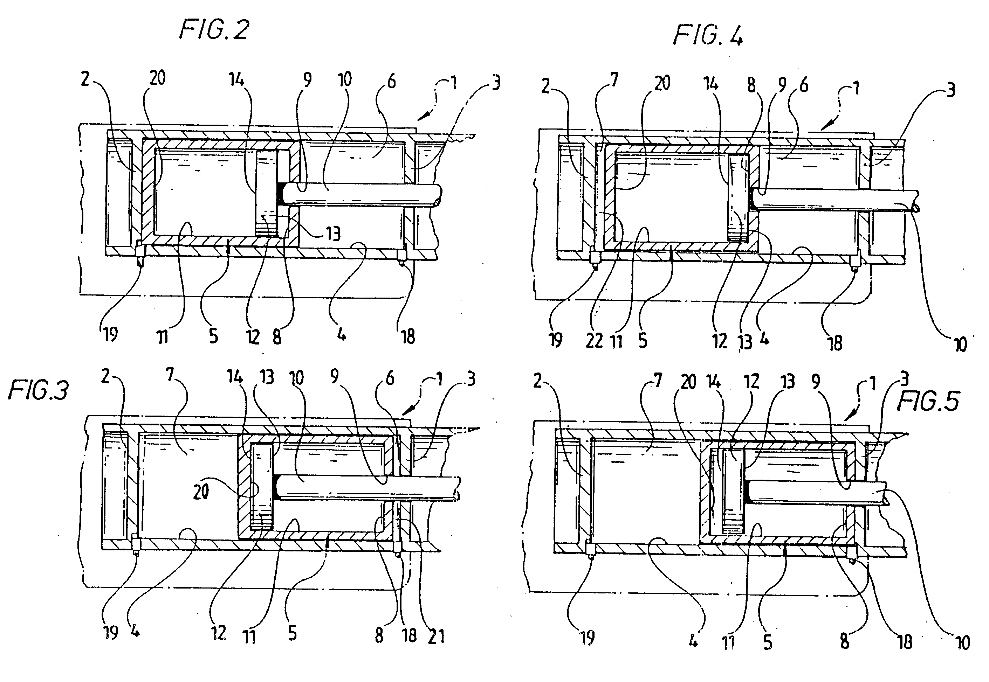

Anhand der Figuren 2 bis 5 ergibt sich, wie die Bedienungsperson wählen kann, in welche Richtung Schläge übertragen werden.

Bei dem Ausführungsbeispiel der Figur 1 ist das Schlagwerkzeug in seiner Ausgangsstellung gezeigt, bei der ein Schlagen zunächst noch nicht möglich ist. Um das Schlagwerkzeug in Arbeitsstellung zu überführen, wird zunächst über die hintere Druckmittelleitung 19 Druckmittel, beispielsweise Druckluft, in die hintere Kammer 7 eingeleitet. Dadurch wird der Kolben 5 nach vorne verschoben und nimmt dabei auch die Werkzeughalterung 15 nach vorne mit, bis die Steuerung des Antriebes den Kolben in die umgekehrte Richtung umsteuert, bis der Kolben wieder an der hinteren Wand 2 anschlägt, wie dies in Figur 2 dargestellt ist. Dabei wird der Ringflansch 12 nicht in die in Figur 1 dargestellte, zurückgezogene Ausgangsstellung mitgenommen, sondern er bleibt - wie in Figur 2 dargestellt - in der vorgeschobenen osition.

Wenn der Kolben durch die Steuerung wieder in Vorwärtsrichtung getrieben wird, trifft daher seine hintere Stirnwand 20 auf die hintere Anschlagfläche 14 des Ringflansches 12 und übt damit einen das Werkzeug in einen Knochen eintreibenden Schlag auf dieses aus, wie in Figur 3 dargestellt ist. Dabei wird der Kolben 5 durch den Anschlag am Ringflansch 12 daran gehindert, bis zur vorderen Wand 3 des Zylinderraumes 4 zu gelangen, das heißt zwischen dem Kolben 5 und der vorderen Wand 3 bleibt ein geringer Spalt 21, die Vorwärtsbewegung des Kolbens 5 wird bei dieser Betriebsart also ausschließlich durch den Ringflansch 12 begrenzt. Dabei kann die Breite des Spaltes 21 unterschiedlich sein und hängt nur davon ab, wie weit der Ringflansch 12 in das Innere des Griffteils 1 eingeschoben ist. Dieses Einschieben kann der Operateur dadurch variieren, daß er den Griffteil 1 mehr oder weniger gegen den Knochen drückt, da der Griffteil gegenüber dem Werkzeug frei verschieblich ist. Dadurch ist es für den Operateur auch möglich, die Größe des Kolbenhubes zu beeinflussen, denn bei großem Spalt 21 legt der Kolben nur einen kleinen Hub zurück und kann daher bei seiner Bewegung nicht so stark beschleunigt werden wie bei einem großen Hub. Der Operateur hat also durch das gefühlvolle Vorschieben des Griffteils gegenüber dem Knochen die Möglichkeit, die Schlagstärke in gewissem Umfange zu variieren.

Wenn der Operateur dagegen den Griffteil 1 vom Knochen 17 wegzieht, wird der Ringflansch 12 in Richtung auf die vordere Wand 3 des Zylinderraums 4 verschoben, da das Werkzeug im Knochen festgehalten wird, Dies führt dazu, daß bei der Rückbewegung der Kolben 5 mit seiner vorderen Stirnwand 8 an den Ringflansch 12 anschlägt, ehe der Kolben an der hinteren Wand 2 des Zylinderraumes 4 anschlagen kann, das heißt es verbleibt nunmehr zwischen Kolben 5 und hinterer Wand 2 des Zylinderraums 4 ein Spalt 22 (Figur 4).

Bei der vorwärtsbewegung des Kolbens schlägt der Kolben bei dieser Stellung des Ringflansches gegen die vordere Wand 3 des Zylinderraumes 4, wobei der Ringflansch 12 nicht getroffen wird (Figur 5), das heißt bei der Vorwärtsbewegung wird kein Schlag auf den Ringflansch und damit den Werkzeughalter übertragen. Der Operateur erreicht also durch Wegziehen des Griffteils vom Knochen, daß nur in Ausziehrichtung Schläge auf den Werkzeughalter übertragen werden. Auch hier kann der Operateur die Wegstrecke kontrollieren, die der Kolben von der vorderen Endstellung (Figur 5) bis zur Anschlagstellung (Figur 4) am Ringflansch zurücklegt, das heißt welche Beschleunigungsstrecke er zurücklegt, indem er die Position des Griffteils 1 relativ zur Werkzeughalterung verändert.

Bei den bisher beschriebenen Ausführungsbeispielen läßt sich der Griffteil 1 gegenüber der Werkzeughalterung beliebig verschieben, so daß der Operateur die Position des Ringflanschs 12 längs des Zylinderraumes 4 frei wählen kann. Es sind auch Ausführungsbeispiele möglich, bei denen die Verschieblichkeit des Werkzeughalters gegenüber dem Griffteil 1 bebeschränkt ist, so daß die Verschiebung nur gerade ausreicht, um eine Umsteuerung der Schlagrichtung vorzunehmen, während eine Variation der freien Beschleunigungsstrecke des Kolbens bis zum Auftreffen auf dem Ringflansch nur noch in geringem Maße möglich ist.

## Patentansprüche

1. Schlagwerkzeug für chirurgische Instrumente mit einem hülsenförmigen Griffteil (1), einem in Längsrichtung in diesem verschieblichen Werkzeughalter (12, 15) und einem im Griffteil (1) in dessen Längsrichtung verschieblichen, oszillierend angetriebenen (5) Kolben, der zwei Schlagflächen (8, 20) aufweist, die an entsprechenden Anlageflächen des Werkzeughalters (13, 14) anschlagen und diesen dabei mit in entgegengesetzter Richtung wirkenden Kraftstößen beaufschlagen,
**dadurch gekennzeichnet,** daß der tatsächliche Hub (H₁) des Kolbens (5) im Griffteil (1) derart beschränkt ist, daß er kleiner ist als der Doppelschlaghub (H₂), den der Kolben (5) bei im Griffteil (1) festgehaltenem Werkzeughalter (12, 15) vom Anschlagen der ersten Schlagfläche (8) an der ersten Anlagefläche (13) bis zum Anschlagen der zweiten Schlagfläche (20) an der zweiten Anlagefläche (14) zurücklegen müßte, und daß der Werkzeughalter (12, 15) sowohl relativ zum Griffteil (1) als auch relativ zum Kolben (5) verschieblich im Griffteil (1) gelagert ist.

2. Schlagwerkzeug nach Anspruch 1, dadurch gekennzeichnet, daß der Kolben (5) einen Innenraum (11) aufweist, in den durch eine Stirnfläche (8) des Kolbens (5) der Werkzeughalter (12, 15) eintritt. daß die in entgegengesetzte Richtung weisenden Anlageflächen (13, 14) des Werkzeughalters (12, 15) im Innenraum (11) des Kolbens (5) angeordnet sind und daß sich auch die Schlagflächen (8, 20) des Kolbens (5) in diesem Innenraum (11) befinden.

3. Schlagwerkzeug nach Anspruch 2, dadurch gekennzeichnet, daß der Werkzeughalter an seinem im Inneren des Kolbens (5) angeordneten Ende einen Ringflansch (12) trägt, dessen gegenüberliegende Stirnflächen die beiden Anlageflächen (13, 14) bilden.

4. Schlagwerkzeug nach Anspruch 3, dadurch gekennzeichnet, daß die den Innenraum (11) des Kolbens (5) begrenzenden Stirnflächen (8, 20) die beiden Schlagflächen bilden.

5. Schlagwerkzeug nach einem der voranstehenden Ansprüche, dadurch gekennzeichnet, daß der maximale Hub (H₁) des Kolbens (5) nur um etwa 1 mm kleiner ist als der Doppelschlaghub (H₂).

6. Schlagwerkzeug nach einem der voranstehenden Ansprüche, dadurch gekennzeichnet, daß der Hubweg des Werkzeughalters (12, 15) durch Anschlagorgane (8, 20) begrenzt ist.

7. Schlagwerkzeug nach einem der voranstehenden Ansprüche, dadurch gekennzeichnet, daß das Werkzeug durch einen Verschluß lösbar mit dem Werkzeughalter verbunden ist.

## Claims

1. A percussion tool for surgical instruments, comprising a sleeve-type handle part (1), a tool holder (12, 15) displaceable therein in the longitudinal direction and a piston (5) which is displaceable in the handle part (1) in the longitudinal direction thereof and driven so as to oscillate and which has two impact surfaces (8, 20) which strike corresponding contact surfaces of the tool holder (13, 14) and act upon the latter with impulses acting in the opposite direction, characterised in that the actual stroke (H₁) of the piston (5) in the handle part (1) is restricted in such a way that it is smaller than the double impact stroke (H₂) which the piston (5) would have to cover from the first impact surface (8) striking the first contact surface (13) to the second impact surface (20) striking the second contact surface (14) if the tool holder (12, 15) were secured in the handle part (1), and in that the tool holder (12, 15) is mounted in the handle part (1) so as to be displaceable relative to both the handle part (1) and the piston (5).

2. A percussion tool according to claim 1, characterised in that the piston (5) has an inner chamber (11) which the tool holder (12, 15) enters through one end surface (8) of the piston (5), in that the contact surfaces (13, 14) of the tool holder (12, 15) are arranged in the inner chamber (11) of the piston (5), the said contact surfaces (13, 14) facing in opposite directions, and in that the impact surfaces (8, 20) of the piston (5) are also located in the said inner chamber (11).

3. A percussion tool according to claim 2, characterised in that the tool holder carries a ring flange (12) on its end arranged in the interior of the piston (5), the opposing end surfaces of the said ring flange (12) forming the two contact surfaces (13, 14).

4. A percussion tool according to claim 3, characterised in that the end surfaces (8, 20) delimiting the inner chamber (11) of the piston (5) form the two impact surfaces.

5. A percussion tool according to any one of the preceding claims, characterised in that the maximum stroke (H₁) of the piston (5) is only approximately 1 mm smaller than the double impact stroke (H₂).

6. A percussion tool according to any one of the preceding claims, characterised in that the travel of the tool holder (12, 15) is restricted by stop elements (8, 20).

7. A percussion tool according to any one of the preceding claims, characterised in that the tool is detachably connected to the tool holder by means of a fastening.

## Revendications

1. Outil de percussion pour instrument chirurgical comprenant une partie de poignée en forme de fourreau (1), un porte-outil (12,15) mobile en direction longitudinale dans cette partie de poignée, et un piston mû de façon oscillante (5) et mobile dans la partie de poignée dans la direction longitudinale de celle-ci, ledit piston présentant deux surfaces de frappe (8, 20) qui viennent frapper des surfaces d'appui correspondantes du porte-outil (13, 14) et appliquent ainsi des forces de choc agissant en direction opposée,
caractérisé en ce que la course effective (H₁) du piston (5) dans la partie de poignée est limitée de telle manière qu'elle soit inférieure à la course de frappe double (H₂) que le piston (5) devrait parcourir lorsque le porte-outil (12, 15) est maintenu fixe dans la partie de poignée (1) depuis le choc de la première surface de frappe (8) contre la première surface d'appui (13) jusqu'au choc de la seconde surface de frappe (20) contre la seconde surface d'appui (14), et en ce que le porte-outil (12, 15) est monté dans la partie de poignée de manière à pouvoir se déplacer aussi bien par rapport à la partie de poignée (1) que par rapport au piston (5).

2. Outil de percussion selon la revendication 1, caractérisé en ce que le piston (5) présente une cavité intérieure (11), dans laquelle le porte-outil (12, 15) pénètre à travers une face d'extrémité (8) du piston (5), en ce que les surfaces d'appui (13, 14), dirigées en direction opposée, du porte-outil (12, 15) sont agencées dans la cavité intérieure (11) du piston (5), et en ce que les surfaces de frappe (6, 20) du piston (5) sont également situées dans cette cavité (11).

3. Outil de percussion selon la revendication 2 caractérisé en ce que le porte-outil porte une bride annulaire (12) à son extrémité agencée à l'intérieur du piston (5), dont les faces d'extrémité opposées forment les surfaces d'appui (13, 14).

4. Outil de percussion selon la revendication 3, caractérisé en ce que les surfaces d'extrémité (8, 20) qui limitent la cavité intérieure (11) du piston (5) forment les deux surfaces de frappe.

5. Outil de percussion selon l'une des revendications précédentes, caractérisé en ce que la course maximale (H₁) du piston (5) est inférieure de seulement environ 1 mm à la course de frappe double (H₂).

6. Outil de percussion selon l'une des revendications précédentes, caractérisé en ce que la course du porte-outil (12, 15) est limitée par des organes de butée (8, 20).

7. Outil de percussion selon l'une des revendications précédentes, caractérisé en ce que l'outil est relié de façon détachable au porte-outil au moyen d'un verrouillage.
